(19) **Europäisches Patentamt**

**European Patent Office**

**Office européen des brevets**

(11) **EP 0 688 826 B2**

(12) **NOUVEAU FASCICULE DE BREVET EUROPEEN**

(45) Date de publication et mention de la décision concernant l'opposition:
**11.01.2006 Bulletin 2006/02**

(45) Mention de la délivrance du brevet:
**26.01.2000 Bulletin 2000/04**

(21) Numéro de dépôt: **95401335.5**

(22) Date de dépôt: **09.06.1995**

(51) Int Cl.:
*C08L 77/12* (2006.01)    *C08L 77/00* (2006.01)
*C08J 5/18* (2006.01)    *B32B 27/12* (2006.01)
*B32B 27/08* (2006.01)

(54) **Film imper-respirant**

Wasserdampfdurchlässiger, wasserdichter Film

Water-resistant vapour-permeable film

(84) Etats contractants désignés:
**AT BE CH DE DK ES FR GB GR IE IT LI LU NL PT SE**

(30) Priorité: **20.06.1994 FR 9407514**

(43) Date de publication de la demande:
**27.12.1995 Bulletin 1995/52**

(73) Titulaire: **Arkema**
**92800 Puteaux (FR)**

(72) Inventeurs:
• **Fischer, Laurent**
**F-27470 Serquigny (FR)**
• **Degrand, Michel,**
**Les Bruyères du Bois Taillefer**
**F-27300 Bernay (FR)**
• **Bouilloux, Alain**
**F-27300 Bernay (FR)**
• **Jammet, Jean-Claude**
**F-27190 Glisolles (FR)**
• **Germain, Yves**
**F-27470 Serquigny (FR)**

(56) Documents cités:
EP-A- 0 046 071        EP-A- 0 378 015
EP-A- 0 459 862        EP-A- 0 560 630
WO-A-95/16476        US-A- 3 326 833
US-A- 3 808 047        US-A- 4 205 158
US-A- 4 739 012        US-A- 5 039 744
US-A- 5 120 813

EP 0 688 826 B2

## Description

La présente invention concerne un film imper-respirant, c'est-à-dire un matériau perméable à la vapeur d'eau et imperméable à l'éau.

La demande de brevet européen EP 0378 015 décrit des films imper-respirants en polyétheresteramide qui peuvent être collés à chaud ou à l'aide d'un adhésif sur du tissu, du cuir ou une matière plastique.

La demande de brevet EP 560 630 décrit des matériaux imper-respirants. Ils sont constitués (exemple 6) de deux films (1) et (2) coextrudés, sur chaque face du film ainsi obtenu, on colle un non tissé à l'aide d'une colle à base de polyuréthanne en solution dans le trichloréthylène.

Le film (1) est un mélange de polyétheresteramide, de polyamide 6 et de polyéthylène greffé anhydride maléique, le film (2) est constitué d'un autre polyétheresteramide. Du côté du film (1), on colle un non tissé à base de polyamide, du côté du film (2), on colle un non tissé en polyester.

L'exemple 7 du même art antérieur décrit le même bicouche constitué des films (1) et (2) coextrudés mais du côté du film (1) on colle un non tissé en polyester/polyéthylène et du côté du film (2) on colle un non tissé en polyester.

On utilise ce matériau pour faire des vêtements en disposant le non tissé polyester/polyéthylène du côté de l'intérieur du vêtement et le non tissé polyester vers l'extérieur du vêtement. Le matériau rend le vêtement perméable à la vapeur d'eau et imperméable à l'eau et au sang qui pourrait être projeté depuis l'extérieur vers le vêtement, protégeant ainsi le porteur du vêtement.

La demande de brevet EP 459 862 décrit des films constitués d'un mélange de polyétheresteramide et d'une polyoléfine modifiée choisie parmi :

- les copolymères de l'éthylène et de l'acétate de vinyle, éventuellement maléisés ;
- les copolymères de l'éthylène et de l'acide (méth)acrylique ;
- les copolymères de l'éthylène, de l'acétate de vinyle et éventuellement de (méth)acrylate d'alkyle ;
- les copolymères de l'éthylène, de (méth)acrylate d'alkyle et éventuellement d'anhydride maléique.

Ces films peuvent être collés sur des tissus et des non tissés.

L'exemple 8-1 montre un film constitué d'un mélange d'un polyétheresteramide et d'un EVA maléisé collé sur un textile.

L'épaisseur des films décrits dans EP 459 862 est de 100 à 500 $\mu$m, ce ne sont pas des films imper-respirants.

Les films imper-respirants de l'art antérieur essentiellement en polyétheresteramides ont l'inconvénient, quand ils sont très perméables, d'avoir une forte reprise d'humidité qui provoque leur gonflement et les rend fragiles.

Les films en alliage de polyétherestéramides et de polyamides ne sont pas assez souples et se collent difficilement sur des non tissés.

On a maintenant trouvé des films imper-respirants en alliages de polyétheresteramide et de copolyacrylates qui ont une forte perméabilité tout en ayant une faible reprise d'humidité. Un deuxième avantage des films de l'invention est qu'ils sont faciles à extruder à grande vitesse et sans défauts, de plus, ils ne bloquent pas et leur aspect est soyeux. Un troisième avantage est qu'ils possèdent un fort allongement avant rupture. Un quatrième avantage est qu'on peut les associer facilement à des non tissés.

La présente invention est donc un film imper-respirant constitué d'un mélange comprenant :

<u>a</u> au moins un élastomère thermoplastique ayant des blocs polyéther dérivés du polyéthylèneglycol;
<u>b</u> au moins un copolymère comprenant de l'éthylène et au moins un (méth)acrylate d'alkyle.

La quantité de a), en poids, est de plus de 50 parties pour 100 parties de (a) et (b).

L'élastomère thermoplastique peut comprendre des motifs polyéthers et des motifs polyesters, ce sont par exemple des blocs polyéthers et des blocs ou des motifs polyesters.

Ces produits sont connus sous le nom de polyesters élastomères et sont thermoplastiques.

La masse molaire $\overline{M}n$ de ces polyéthers peut être comprise entre 250 et 6000.

Les segments souples des polyesters élastomères sont formés par les motifs polyéthers précédents et au moins un diacide carboxylique tel que par exemple l'acide téréphtalique.

Les segments rigides des polyesters élastomères comprennent des motifs glycol, propanediol ou butane diol-1,4 et des motifs diacides carboxyliques reliés par des fonctions esters. Les diacides carboxyliques peuvent être les mêmes que les précédents.

Les segments rigides peuvent comprendre plusieurs motifs résultant de l'action d'un diol sur un diacide.

Les segments souples peuvent comprendre plusieurs motifs résultant de l'action du polyéther sur un diacide. Les segments durs et les segments souples sont attachés par des liaisons ester. De tels polyesters élastomères sont décrits dans les brevets EP 402 883 et EP 405 227.

Les élastomères thermoplastiques à motifs polyéthers peuvent aussi être des copolyétherimides ester. Les segments souples sont formés par la réaction de polyéther diamines avec des composés tricarboxyliques ou anhydrides d'acide carboxylique contenant un groupe carboxylique tel que par exemple l'anhydride trimellitic. Les polyéthers diamines utilisés ont une masse molaire moyenne de 600 à 12000. Ces polyétherdiamines peuvent eux-mêmes être issus de polyéthylèneglycol.

Les blocs polyesters formant les segments durs dans des copolyétherimides ester résultent par exemple de la condensation d'au moins un diol avec au moins un diacide carboxylique. Le diol peut être le glycol, le propanediol ou le butanediol. Le diacide peut être l'acide téréphtalique. De tels copolyétherimides ester sont décrits dans EP 402 883 et EP 405 227.

Les élastomères thermoplastiques à blocs polyéther peuvent être aussi des polyétheruréthannes. Ils sont formés par l'enchaînement de trois composants de base :

(i) un polyétherdiol tel que un polyéthylène glycol. La masse molaire peut être de 500 à 6 000 ;
(ii) un diisocyanate tel qu'un MDI ou un TDI ;
(iii) un diol de faible masse tel que le glycol (éthane diol), le butane diol-1,4, ou le 1,4-phénylène-bis-β-hydroxyéthyl éther comme allongeur de chaîne.

Les élastomères (a) à blocs polyéther peuvent être aussi des polymères à blocs polyamides et blocs polyéther.

Les polymères à blocs polyamides et blocs polyéthers résultent de la copolycondensation de séquences polyamides à extrémités réactives avec des séquences polyéthers à extrémités réactives, telles que, entre autres :

1) Séquences polyamides à bouts de chaîne diamines avec des séquences polyoxyalkylènes à bouts de chaînes dicarboxyliques.
2) Séquences polyamides à bouts de chaînes dicarboxyliques avec des séquences polyoxyalkylènes à bouts de chaînes diamines obtenues par cyanoéthylation et hydrogénation de séquences polyoxyalkylène alpha-oméga dihydroxylées aliphatique appelées polyétherdiols.
3) Séquences polyamides à bouts de chaînes dicarboxyliques avec des polyétherdiols, les produits obtenus étant, dans ce cas particulier, des polyétheresteramides.

Les séquences polyamides à bouts de chaînes dicarboxyliques proviennent, par exemple, de la condensation d'acides alpha-oméga aminocarboxyliques de lactames ou de diacides carboxyliques et diamines en présence d'un diacide carboxylique limiteur de chaîne. Avantageusement, les blocs polyamides sont en polyamide-12.

La masse molaire en nombre $\overline{M}_n$ des séquences polyamides est comprise entre 300 et 15 000 et de préférence entre 600 et 5 000. La masse $\overline{M}_n$ des séquences polyéther est comprise entre 100 et 6 000 et de préférence entre 200 et 3 000.

Les polymères à blocs polyamides et blocs polyéthers peuvent aussi comprendre des motifs répartis de façon aléatoire. Ces polymères peuvent être préparés par la réaction simultanée du polyéther et des précurseurs des blocs polyamides.

Par exemple, on peut faire réagir du polyétherdiol, un lactame (ou un alpha-oméga amino acide) et un diacide limiteur de chaîne en présence d'un peu d'eau. On obtient un polymère ayant essentiellement des blocs polyéthers, des blocs polyamides de longueur très variable, mais aussi les différents réactifs ayant réagi de façon aléatoire qui sont répartis de façon statistique le long de la chaîne polymère.

Ces polymères à blocs polyamides et blocs polyéthers qu'ils proviennent de la copolycondensation de séquences polyamides et polyéthers préparées auparavant ou d'une réaction en une étape présentent, par exemple, des duretés shore D pouvant être comprises entre 20 et 75 et avantageusement entre 30 et 70 et une viscosité intrinsèque entre 0,8 et 2,5 mesurée dans le métacrésol à 250° C pour une concentration initiale de 0,8 g/100 ml.

Les blocs polyéthers dérivent du polyéthylène glycol, ils sont soit utilisés tels quels et copolycondensés avec des blocs polyamides à extrémités carboxyliques, soit ils sont aminés pour être transformés en polyéther diamines et condensés avec des blocs polyamides à extrémités carboxyliques. Ils peuvent être aussi mélangés avec des précurseurs de polyamide et un limiteur de chaîne pour faire les polymères à blocs polyamides et blocs polyéthers ayant des motifs répartis de façon statistique.

Des polymères à blocs polyamides et polyéthers sont décrits dans les brevets US 4 331 786, US 4 115 475, US 4 195 015, US 4 839 441, US 4 864 014, US 4 230 838 et US 4 332 920.

Que les blocs polyéther soient dans la chaîne du polymère à blocs polyamides et blocs polyéther sous forme de diols ou de diamines, on les appelle par simplification blocs PEG.

De préférence, le polymère à blocs polyamides et blocs polyéther comprend un seul type de bloc polyamide et un seul type de blocs polyéther. On utilise avantageusement des polymères à blocs PA-12 et des polymères à blocs PA-6.

On peut aussi utiliser un mélange de deux ou plusieurs polymères à blocs polyamides et blocs potyéther.

Avantageusement, le polymère à blocs polyamides et blocs polyéthers est tel que le polyamide est le constituant majoritaire en poids, c'est-à-dire que la quantité de polyamide qui est sous forme de blocs et celle qui est éventuellement répartie de façon statistique dans la chaîne représente 40 % en poids ou plus du polymère à blocs polyamide et blocs polyéther. Avantageusement, la quantité de polyamide et la quantité de polyéther sont dans le rapport (polyamide/polyéther) 1/1 à 3/1 et de préférence :

l'élastomère (a) peut être un mélange de deux ou plusieurs des élastomères à blocs polyéthers cités plus haut, c'est-à-dire que (a) peut être un mélange de deux polyétheruréthannes, ou d'un polyétheruréthanne et d'un polyétheresteramides ou toute autre combinaison.

La perméabilité à la vapeur d'eau varie avec la quantité de blocs polyéther de (a) et avec la nature de ces blocs. Le PEG est très perméable à la vapeur d'eau.

Le groupement alkyle du (méth)acrylate d'alkyle entrant dans le copolymère b peut avoir jusqu'à 10 atomes de carbone et peut être linéaire, ramifié ou cyclique. A titre d'illustration du (méth)acrylate d'alkyle, on peut citer notamment l'acrylate de n-butyle, l'acrylate d'isobutyle, l'acrylate d'éthyl-2 hexyle, l'acrylate de cyclohexyle, le méthacrylate de méthyle, le méthacrylate d'éthyle. Parmi ces (méth)acrylates, on préfère l'acrylate d'éthyle, l'acrylate de n-butyle et le méthacrylate de méthyle.

Selon une forme particulière de l'invention le copolymère (b) peut être greffé ou copolymérisé avec (i) un acide carboxylique insaturé tel que par exemple l'acide (méth)acrylique, (ii) un anhydride d'acide carboxylique insaturé tel que par exemple l'anhydride maléique ou (iii) un époxyde insaturé tel que par exemple le (méth)acrylate de glycidyle.

(b) peut être un mélange d'au moins deux de ces copolymères.

Selon une autre forme de l'invention, (b) est un mélange d'un polymère (b1) et d'un polymère (b2).

(b2) est un copolymère comprenant de l'éthylène et un (méth)acrylate d'alkyle. Le (méth)acrylate d'alkyle peut être celui défini plus haut.

(b1) est différent de (b2) et est choisi parmi :

- les polyoléfines homo ou copolymères éventuellement greffées, tels que par exemple le polyéthylène, le polypropylène, les copolymères de l'éthylène avec des alphaoléfines. Ces produits pouvant être greffés par des anhydrides d'acides carboxyliques insaturés tels que l'anhydride maléique ou des époxydes insaturés tels que le méthacrylate de glycidyle.
- les copolymères de l'éthylène avec au moins un produit choisi parmi (i) les acides carboxyliques insaturés, leurs sels, leurs esters, (ii) les esters vinyliques d'acides carboxyliques saturés, (iii) les acides dicarboxyliques insaturés, leurs sels, leurs esters, leurs hemiesters, leurs anhydrides (iv) des époxydes insaturés.

Ces copolymères de l'éthylène pouvant être greffés par des anhydrides d'acides dicarboxyliques insaturés ou des époxydes insaturés.

- les copolymères blocs styrène / butadiène / styrène (SBS), styrène 1 isoprène / styrène (SIS) ou styrène / éthylène-butène / styrène (SEBS), ces copolymères pouvant être greffés par un anhydride d'acide carboxylique insaturé tel que l'anhydride maléique.

Avantageusement, les proportions de (a) et (b) sont telles que le mélange se présente sous forme d'une matrice dé (a) dans taquette (b) est dispersé par exemple sous forme de nodules. Ces proportions dépendent de la nature de (a) ainsi que de la nature de (b) et en particulier des éventuelles fonctions acides, anhydrides ou époxydes présentes dans (b).

La quantité de a est de plus de 50 parties pour 100 parties de (a) et (b), de préférence de 55 à 80 parties. (Il s'agit de parties en poids, et ainsi dans la suite du texte).

On utilise de préférence comme élastomère a un copolymère ayant des blocs polyamides reliés à des blocs polyéther, la liaison se fait par des liaisons esters (polyétheresteramides).

On peut l'obtenir par condensation de blocs polyamides à extrémités acides avec des blocs polyéther à extrémités OH.

On préfère les polyamides 6 et 12 et les polyéthylèneglycols (PEG).

Avantageusement, (b1) est un copolymère comprenant de l'éthylène, au moins un (méth)acrylate d'alkyle et un monomère insaturé ayant une fonction acide ou anhydride d'acide carboxylique.

Le monomère insaturé de b1 ayant une fonction acide ou anhydride peut être :

- un anhydride d'acide dicarboxylique insaturé tel que l'anhydride citraconique, l'anhydride itaconique, l'anhydride tétrahydrophtalique, l'anhydride méthyl-2 maléique, l'anhydride diméthyl-2,3 maléique et l'anhydride maléique ;
- un acide ou diacide insaturé tel que l'acide (méth)acrylique, l'acide crotonique ou l'acide cinnamique ;

Le copolymère b1 est avantageusement un copolymère éthylène - (méth)acrylate d'alkyle -anhydride maléique contenant en poids 2 à 10 % d'anhydride maléique et au moins 50 % d'éthylène.

Le (méth)acrylate d'alkyle de b1 peut être choisi dans la même famille que les (méth)acrylates de b2.

Le copolymère b2 contient avantageusement 5 à 40 % en poids de (méth)acrylate d'alkyle et de préférence 20 à 35 %.

Avantageusement, le film thermoplastique de l'invention comprend :

a) un polymère à blocs polyamide et blocs polyéther PEG, le polyamide étant de préférence du PA-12 ;
b) un copolymère de l'éthylène et d'un (méth)acrylate d'alkyle ;
c) un copolymère éthylène / (méth)acrylate d'alkyle / anhydride maléique.

Les proportions préférées sont de :

60 à 80 parties de a)
20 à 30 parties de b)
et 5 à 40 parties de c)

Ces films ont une épaisseur pouvant être par exemple entre 10 et 80 $\mu$m et de préférence de 15 à 35 $\mu$m.

Le mélange de polymères qui constitue le film imper-respirant de l'invention peut éventuellement contenir des charges organiques et/ou minérales. A titre d'exemple de charges, on peut citer notamment la silice, l'oxyde de titane. Le mélange peut également contenir divers additifs tels que agents anti UV, agents démoulants, modifiants chocs...., ainsi que des colorants ou des pigments.

On fabrique ces mélanges par les techniques usuelles de mélange à l'état fondu, puis on les met en film par les techniques connues en soi.

Les films de l'invention sont imperméables à l'eau et aux solutions aqueuses, ils sont perméables à la vapeur d'eau et ne sont pas microporeux, c'est-à-dire que ce sont des films continus. Ils ont une bonne perméabilité à la vapeur d'eau, pouvant atteindre 25 000 g/m$^2$/24 h selon la norme ASTM E 96 BW (films en contact avec l'eau).

Ils ont une reprise d'humidité très nettement inférieure à celle d'un film constitué essentiellement d'un polymère comprenant des blocs PEG.

Ils peuvent être préparés par extrusion à grande vitesse. Les films ne sont pas bloquants, ils sont souples, d'un toucher soyeux, et ne sont pas bruyants.

Ces films sont utiles pour faire des pansements, des patches, des poches pour ostomy, des gants.

L'invention concerne aussi ces objets.

Les films de l'invention peuvent être fixés sur un tissé ou sur un non tissé essentiellement sans adhésif par exemple par couchage à chaud ou par pressage. On peut aussi utiliser des adhésifs soit en couche complète entre le film imper-respirant et le non tissé soit en bandes ou toute application discontinue, telle que des pointillés ou des taches.

Ces adhésifs peuvent être des hot melts.

La présente invention concerne aussi ces matériaux imper respirants formés du film de l'invention associé à un tissé ou à un non tissé. On ne sortirait pas du cadre de l'invention en ajoutant d'autres couches, soit du côté du film imper respirant, soit du côté du tissé ou non tissé.

Ces autres couches pouvant être des tissés ou non tissés identiques ou différents de ceux déjà présents.

Ces matériaux imper respirants formés du film imper respirant associé à un tissé ou un non tissé sont souples et comme le film a une faible reprise d'humidité, il se délamine moins facilement du tissé ou du non tissé qu'un film constitué seulement de polymère à blocs polyéther. Ces matériaux sont utiles pour faire des vêtements de protection du personnel médical, des articles d'hygiène jetables, des alèzes de matelas, des films sous toiture pour les maisons, des vêtements, des chaussures.

### *Exemples*

Produit (A) : soit un polyétheresteramide constitué de séquences bloc de Polyamide 12 $\overline{M}_n$ = 1 500 et de séquences bloc de polyétherglycol (PEG) ($\overline{M}_n$ = 1 500).

La viscosité intrinsèque mesurée à 20° C dans le métacrésol est 1,45 à 1,60.

Produit (B) : soit un polyétheresteramide constitué de séquences de polyamide 12 ($\overline{M_n}$ = 4 500) et de séquences blocs de polyétherglycol (PEG) ($\overline{M_n}$ = 1 500).

La viscosité intrinsèque mesurée à 20° C dans le métacrésol est 1,4 à 1,55.

Produit (C) : soit un polyétheresteramide constitué de séquence bloc de polyamide 6 ($\overline{M_n}$ = 1 500) et de séquences bloc de polyétherglycol (PEG) ($\overline{M_n}$ = 1 500).

Produit (D) : soit un copolymère d'éthylène-ester acrylique contenant 24 % en poids d'acrylate de méthyle.

Produit (E) : soit un copolymère d'éthylène-ester acrylique contenant 28 % en poids d'acrylate de méthyle.

Produit (F) : soit un terpolymère d'éthylène-ester acrylique - anhydride maléique contenant 19 % en poids de comonomère et 3 % en poids d'anhydride maléique.

On réalise les mélanges suivants sur BUSS PR 46/70 11 D et l'on extrude dans des conditions standards adaptées aux produits des films de 25 μm.

|  | Produit (A) | Produit (B) | Produit (C) | Produit (D) | Produit (E) | Produit (F) |
|---|---|---|---|---|---|---|
| **Mélange①** | 60 |  |  | 30 |  | 10 |
| **Mélange②** |  | 60 |  | 30 |  | 10 |
| **Mélange③ (comparatif)** |  |  | 20 |  | 70 | 10 |
| **Mélange④** | 65 |  |  |  |  | 35 |

On mesure la perméabilité à la vapeur d'eau selon la méthode décrite dans la norme ASTM E 96 méthode BW (film en contact avec l'eau) dans une étuve Heraous Votsch des conditions de température = 38° C et d'humidité relative ambiante = 50 % maintenus durant toute la durée de la mesure.

Le résultat de perméabilité à la vapeur d'eau est donné en g/m$^2$/24 h.

Les imprécisions sur la mesure donnent un résultat à plus ou moins 10 à 20 %.

On mesure la reprise d'humidité des produits par la reprise en poids de granulés trempés 24 h dans l'eau à 20° C préalablement conditionnés dans une atmosphère de 20° C à 65 % d'humidité relative pendant 15 jours.

On évalue l'extrudabilité des produits en mesurant la vitesse maximale d'étirage pour réaliser un film de 25 μm sans avoir de phénomène de résonance des bords du film en sortie d'extrudeuse (necking).

On évalue qualitativement le blocage du film enroulé après extrusion par la facilité de séparer 2 films enroulés.

On évalue le toucher agréable du film à la main.

On évalue le bruit du film quand on le froisse ; le résultat "Bon" étant l'absence de bruit.

On obtient le tableau suivant :

| Produits ou Mélanges | Perméabilité à la vapeur d'eau g/m$^2$/ 24 h | Reprise d'humidité (%) | Extrudabilité (en m/mn) | Blocage du film (*) | Toucher (*) | Bruit (*) |
|---|---|---|---|---|---|---|
| (A) | 23 000 | 50 | ≦ 50 | - | 0 | 0 |
| (B) | 12 000 | 12 | ≦ 60 | + | 0 | - |
| (C) | ≧ 20 000 | 120 |  |  |  | 0 |
| (D) ou (E) | ≦ 350 | ≦ 2 | ≧ 100 | + | + | + |
| ① | 22 000 | 27 | ≧ 100 | + | + | + |
| ② | 11 000 | 8 | ≧ 100 | + | + | + |
| Comparatif ③ | 300 | 5,5 |  |  |  | + |

Suite de tableau

| Produits ou Mélanges | Perméabilité à la vapeur d'eau g/m²/ 24 h | Reprise d'humidité (%) | Extrudabilité (en m/mn) | Blocage du film (*) | Toucher (*) | Bruit (*) |
|---|---|---|---|---|---|---|
| ④ | 22 000 | 27 | $\geqq 100$ | 0 | 0 | + |

| (*) (+) Bon |
|---|
| (0) Moyen |
| (-) Mauvais |

On mesure le module en traction suivant la norme ASTM D 882 (Bandelette I = 15 mm, v = 10 mm/mn, $I_o$ = 100 mn) sur les films en question dans le sens perpendiculaire et le sens parallèle à l'extrusion.

| Produit | (A) | ① | (B) | ② |
|---|---|---|---|---|
| *Sens parallèle à l'extrusion* | | | | |
| Module | 79 MPa | 46 MPa | 235 MPa | 189 MPa |
| Contrainte rupture | 30 MPa | 29 MPa | 50 MPa | 61 MPa |
| Allongement rupture | $\geqq 600\ \%$ | $\geqq 600\ \%$ | $\geqq 500\ \%$ | $\geqq 500\ \%$ |
| *Sens perpendiculaire à l'extrusion* | | | | |
| Module | 80 MPa | 42 MPa | 230 MPa | 162 MPa |
| Contrainte rupture | 22 MPa | 23 MPa | 43 MPa | 38 MPa |
| Allongement rupture | $\geqq 600\ \%$ | $\geqq 600\ \%$ | $\geqq 600\ \%$ | $\geqq 600\ \%$ |

On observe que :

Mélange ①

- Conservation de la perméabilité à la vapeur d'eau par rapport à (A) ;
- Diminution de près de la moitié de la reprise en humidité par rapport à (A) ;
- Augmentation de la vitesse d'extrudabilité par rapport à (A) ;
- Toucher meilleur par rapport à (A) ;
- Beaucoup moins de blocage par rapport à (A) ;
- Meilleure adhésion sur non tissé PP ou PE par rapport à (A).
- Conservation des propriétés mécaniques en rupture (Allongement, contrainte), mais très nette diminution du module du film. Le film est plus souple, moins bruyant, le toucher est soyeux.

Mélange② Idem que exemple① par rapport à (B).

Mélange③ Pas d'amélioration de la perméabilité par rapport à (E).

Mélange④ Idem que exemple① avec moins d'amélioration du blocage et du toucher.

## Revendications

**1.** Film imper-respirant constitué d'un mélange comprenant :

    a) au moins un élastomère thermoplastique ayant des blocs polyéther dérivés du polyéthylène glycol,
    b) au moins un copolymère comprenant de l'éthylène et au moins un (méth)acrylate d'alkyle,

la quantité de a), en poids, est de plus de 50 parties pour 100 parties de (a) et (b).

**2.** Film selon la revendication 1 dans lequel (b) est greffé ou copolymérisé avec (i) un acide carboxylique insaturé, (ii) un anhydride d'acide carboxylique insaturé ou (iii) un époxyde insaturé.

**3.** Film selon la revendication 1 dans lequel (b) est un mélange de (b1) et (b2) dans lequel :

(b2) est un copolymère comprenant de l'éthylène et d'un (méth)acrylate d'alkyle,
(b1) est différent de (b2) et est choisi parmi :

➢ les polyoléfines homo ou copolymères éventuellement greffées ;
➢ les copolymères de l'éthylène avec au moins un produit choisi parmi (i) les acides carboxyliques insaturés, leurs sels, leurs esters, (iii) les acides dicarboxyliques insaturés, leurs sels, leurs esters, leurs hemiesters, leurs anhydrides (iv) les epoxydes insaturés ;
➢ les copolymères blocs SBS (styrène/butadiène/styrène), SIS (styrène/isoprène/styrène), SEBS (styrène/ethylène-butène/styrène) éventuellement greffés.

**4.** Film selon la revendication 3 dans lequel (b1) est un copolymère comprenant de l'éthylène, au moins un (méth)acrylate d'alkyle et un monomère insaturé ayant une fonction acide ou anhydride d'acide carboxylique.

**5.** Film selon l'une des revendications 1 à 4 dans lequel (a) est un copolymère ayant des blocs polyamide et des blocs polyéther.

**6.** Matériau imper-respirant comprenant le film selon l'une des revendications 1 à 5 associé à un non tissé ou à un tissé.

**Patentansprüche**

**1.** Wasserdichter atmungsfähiger Film, bestehend aus einer Mischung, die

a) mindestens ein thermoplastisches Elastomer mit Polyetherblöcken, die sich von Polyethylenglykol ableiten, und
b) mindestens ein Copolymer, das Ethylen und mindestens ein Alkyl(meth)acrylat enthält,

enthält, wobei sich die Menge an a) auf mehr als 50 Gewichtsteile, bezogen auf 100 Gewichtsteile (a) und (b), beläuft.

**2.** Film nach Anspruch 1, bei dem (b) mit (i) einer ungesättigten Carbonsäure, (ii) einem ungesättigten Carbonsäureanhydrid oder (iii) einem ungesättigten Epoxid gepfropft oder copolymerisiert ist.

**3.** Film nach Anspruch 1, bei dem es sich bei (b) um eine Mischung von (b1) und (b2) handelt, in welcher:

(b2) ein Copolymer, das Ethylen und mindestens ein Alkyl(meth)acrylat enthält, ist und
(b1) von (b2) verschieden ist und unter:

- gegebenenfalls gepfropften Polyolefin-Homopolymeren oder -Copolymeren,
- Copolymeren von Ethylen mit mindestens einem unter (i) ungesättigten Carbonsäuren, deren Salzen und deren Estern, (iii) ungesättigten Dicarbonsäuren, deren Salzen, deren Estern, deren Halbestern und deren Anyhdriden und (iv) ungesättigten Epoxiden ausgewählten Produkt und
- gegebenenfalls gepfropften SBS-Blockcopolymeren (SBS = Styrol/Butadien/Styrol), SIS-Blockcopolymeren (SIS = Styrol/Isopren/Styrol) und SEBS-Blockcopolymeren (SEBS = Styrol/Ethylen-Buten/Styrol)

ausgewählt ist.

**4.** Film nach Anspruch 3, bei dem es sich bei (b1) um ein Copolymer, das Ethylen, mindestens ein Alkyl(meth)acrylat und ein ungesättigtes Monomer mit einer Säure- oder Carbonsäureanhydridfunktion handelt.

**5.** Film nach einem der Ansprüche 1 bis 4, bei dem es sich bei (a) um ein Copolymer mit Polyamidblöcken und Polyetherblöcken handelt.

**6.** Wasserdichtes atmungsfähiges Material, das den Film nach einem der Ansprüche 1 bis 5 in Kombination mit einem Vliesstoff oder einem Gewebe enthält.

**Claims**

1. Breathable film consisting of a blend comprising:

   a) at least one thermoplastic elastomer having polyether blocks that are derived from polyethylene glycol;
   b) at least one copolymer comprising ethylene and at least one alkyl (meth)acrylate,

   the quantity of a), by weight, is more than 50 parts per 100 parts of (a) and (b).

2. Film according to Claim 1, in which (b) is grafted or copolymerized with (i) an unsaturated carboxylic acid, (ii) an unsaturated carboxylic acid anhydride or (iii) an unsaturated epoxide.

3. Film according to Claim 1, in which (b) is a blend of (b1) and (b2), in which:

   (b2) is a copolymer comprising ethylene and an alkyl (meth)acrylate,
   (b1) is different from (b2) and is chosen from:

   ▶ optionally grafted polyolefin homopolymers or copolymers;
   ▶ copolymers of ethylene with at least one product chosen from (i) unsaturated carboxylic acids, their salts, their esters; (ii) unsaturated dicarboxylic acids, their salts, their esters, their monoesters, their anhydrides; (iii) unsaturated epoxides; and
   ▶ optionally grafted SBS (styrene/butadiene/ styrene), SIS (styrene/isoprene/styrene) or SEBS (styrene/ethylene-butene/styrene) block copolymers.

4. Film according to Claim 3, in which (b1) is a copolymer comprising ethylene, at least one alkyl (meth)acrylate and an unsaturated monomer having a carboxylic acid or acid anhydride functional group.

5. Film according to one of Claims 1 to 4, in which (a) is a copolymer having polyamide blocks and polyether blocks.

6. Breathable material comprising the film according to one of Claims 1 to 5, used in combination with a nonwoven or a woven.